# FASCICULE DE BREVET EUROPEEN

(11) **EP 0 892 650 B1**
(45) Date de publication et mention de la délivrance du brevet: **10.11.1999**
(21) Numéro de dépôt: 97918206.0
(22) Date de dépôt: 11.04.1997
(51) Int. Cl.: A61M 5/32, A61M 25/06

(54) **DISPOSITIF A CANULE HYPODERMIQUE SOUPLE**
HYPODERMISCHE KANÜLE AUS WEICHEM MATERIAL
FLEXIBLE HYPODERMIC INJECTION CANNULA DEVICE

(30) Priorité: 12.04.1996 FR 9604820
(43) Date de publication de la demande: 27.01.1999
(73) Titulaire: Prodimed, S.A., 60530 Neuilly-en-Thelle (FR)
(72) Inventeur: CAMUS, Michel, F-59175 Templemars (FR)
(74) Mandataire: Ecrepont, Robert
(86) Numéro de dépôt international: FR9700646
(87) Numéro de publication internationale: WO9738745

(56) Documents cités:
- DE-A- 4 038 952
- GB-A- 791 721
- US-A- 3 515 137
- US-A- 4 468 224
- US-A- 5 312 375

## Description

L'invention concerne un dispositif à canule hypodermique, c'est à dire, un dispositif pour l'injection d'un liquide dans un milieu de réception, tel un tissu vivant ou un site implanté dans un tel tissu, voire pour le prélèvement d'un liquide dans un milieu de prélèvement du type précité.

Par canule, on désigne un corps à paroi tubulaire qui comprend notamment un canal axial et deux extrémités opposées dont l'une, dite proximale, est destinée à être raccordée à un instrument et l'autre dite, distale, est destinée à être engagée dans un milieu de réception (voir en particulier le document US-A-5312372).

Cette partie distale des canules est à l'origine de nombreuses piqûres accidentelles de personnes qui manipulent des canules hypodermiques.

De tels accidents interviennent essentiellement lors du retrait d'une canule d'un milieu d'injection ou de prélèvement.

Particulièrement, l'extraction d'une canule du septum d'un site implanté oppose une résistance notable et pour contrer cette résistance qui tend à déplacer le site, la personne qui extrait la canule appuie généralement sur la peau du patient avec le pouce et l'index d'une main pendant qu'avec son autre main, elle effectue l'extraction recherchée.

L'extraction est généralement obtenue soudainement et un effet de rebond dû notamment à la contraction musculaire de la personne qui effectue l'opération induit un mouvement de retour de la canule vers la peau du patient et donc vers les doigts placés en appui autour de la canule.

Un résultat que l'invention vise à obtenir est la prévention des piqûres accidentelles de personnes qui manipulent des canules hypodermiques.

A cet effet, l'invention a pour objet un dispositif à canule hypodermique, c'est à dire, un dispositif pour l'injection d'un liquide dans un milieu de réception, tel un tissu vivant et/ou un site implanté dans un tel tissu, voire pour le prélèvement d'un liquide dans un milieu de prélèvement du type précité, la canule consistant en un corps à paroi tubulaire qui comprend notamment un canal axial et deux extrémités opposées dont l'une, dite proximale, est destinée à être raccordée à un instrument, tel un instrument de perfusion, et l'autre dite, distale, est biseautée et destinée à être engagée dans le milieu de réception.

Dans ce dispositif, le corps tubulaire de la canule est réalisé dans un matériau choisi pour que la canule ait une flexibilité telle que son introduction précise dans un milieu de réception ne peut être obtenue qu'après rigidification au moyen d'un corps rigide, tel une aiguille, introduit dans son canal axial.

Ce dispositif est notamment caractérisé en ce que l'extrémité distale biseautée de la canule est localement façonnée de manière à présenter une surface frontale dont les génératrices d'enveloppe sont sensiblement parallèles à un plan sécant à l'axe longitudinal de ladite canule.

L'invention sera bien comprise à l'aide de la description ci-après faite à titre d'exemple non limitatif, en regard du dessin ci-annexé qui représente :

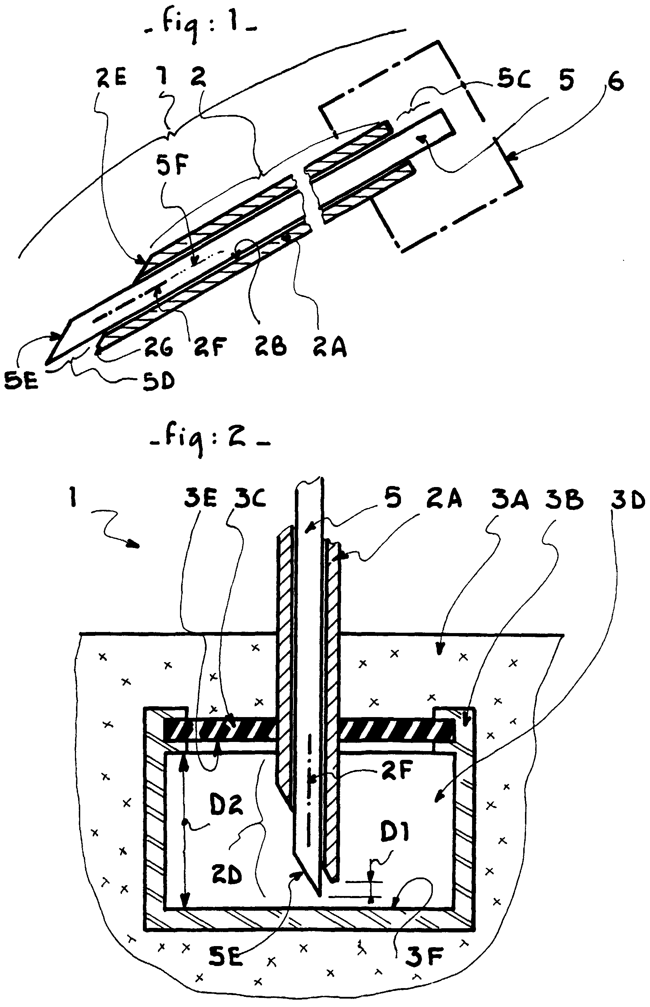

- figure 1 : une vue en coupe longitudinale d'un dispositif selon l'invention,
- figure 2 : une vue partielle et en coupe longitudinale d'un dispositif, selon l'invention, introduit dans un site implanté,

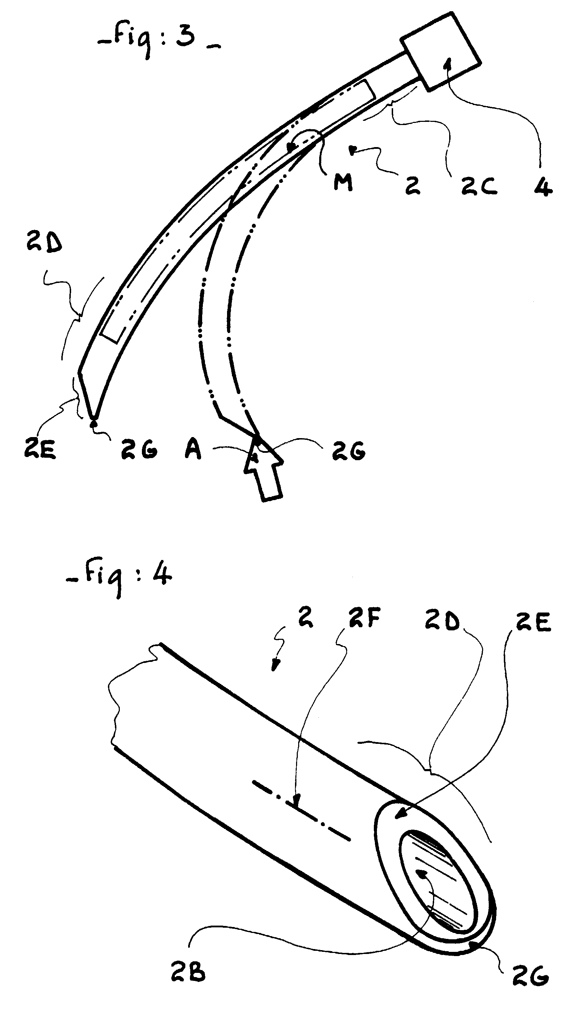

- figure 3 : à plus grande échelle, une vue partielle d'un moyen équipant la canule du dispositif de l'invention,
- figure 4 : à plus grande échelle, une vue partielle et en perspective de la canule du dispositif de l'invention,

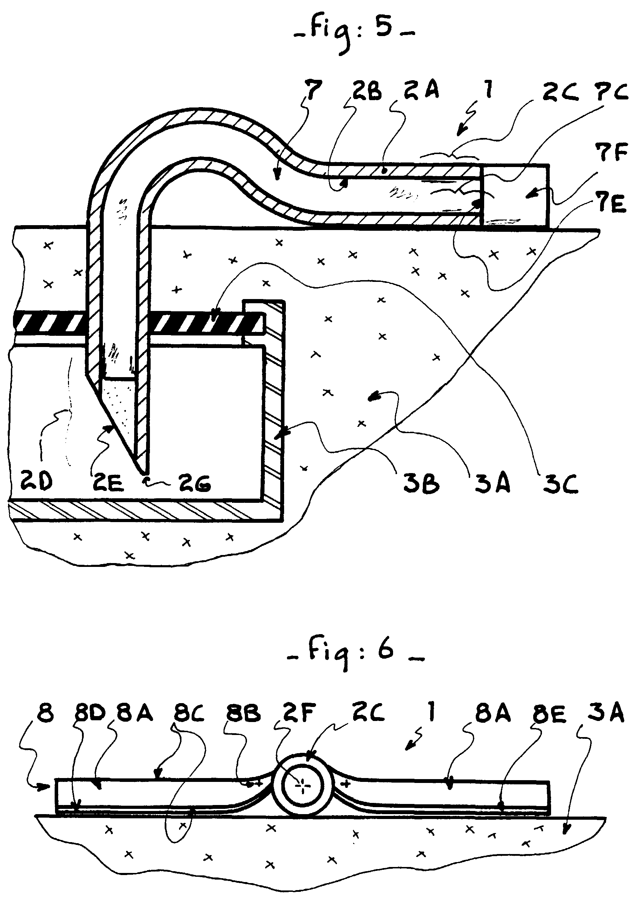

- figure 5 : une vue en coupe partielle longitudinale de la canule du dispositif de l'invention en cours d'utilisation,
- figure 6 : une vue axiale de l'extrémité proximale de la canule du dispositif de l'invention.

En se reportant au dessin, on voit un dispositif 1 à canule hypodermique 2, c'est à dire, un dispositif pour l'injection d'un liquide (non représenté) dans un milieu de réception 3A, 3B, tel un tissu vivant 3A et/ou un site 3B implanté dans un tel tissu 3A, voire pour le prélèvement d'un liquide dans un milieu de prélèvement 3A, 3B du type précité.

La canule 2 consiste en un corps à paroi tubulaire 2A qui comprend notamment un canal axial 2B et deux extrémités opposées 2C, 2D dont l'une 2C, dite proximale, est destinée à être raccordée à un instrument 4 et, l'autre 2D dite, distale, est biseautée et destinée à être engagée dans le milieu 3A, 3B de réception.

Par extrémité biseautée, on désigne une extrémité 2D dont la face terminale 2E est constituée dans un plan sensiblement sécant à un axe longitudinal 2F à la canule 2.

Le corps 2 tubulaire de la canule 2 est réalisé dans un matériau choisi de manière que la canule 2 ait une flexibilité telle que son introduction précise dans un milieu de réception 3A, 3B ne puisse être obtenue qu'après rigidification au moyen d'un corps rigide 5 plein, tel une aiguille, introduit dans son canal axial 2B.

Le fait que la canule 2 ait la flexibilité précitée n'exclut pas que la partie distale biseautée soit réalisée en matériau dûr et rigide.

L'utilisation d'un corps rigide 5 plein permet d'éviter le carottage du milieu traversé et donc du septum.

Le diamètre du corps rigide 5 est ajusté de manière que son retrait de la canule s'effectue sans à-coup.

L'état de la technique comprend de nombreux dispositifs (voir, par exemple, les documents relatifs aux dispositifs hypodermiques qui mettent en oeuvre une canule possédant une telle flexibilité US-A-4.250.881, US-A-4.569.675, EP-A-0.415.653, EP-A-0.564.797, EP-A-0.615.768).

Les canules de ces dispositifs hypodermiques (non représentés) ont leur extrémité distale qui est, soit sensiblement conique (US-A-4.250.881, US-A-4.569.675, EP-A-0.615.768), soit à face perpendiculaire à l'axe longitudinal de la canule (EP-A-0.415.653, US-A-5.135.502).

De telles formes d'extrémité distales permettent de réduire notablement le risque de piqûres accidentelles lorsque la canule a été libérée du corps rigide qui permet son introduction dans un milieu de réception mais, de ce fait, elles s'opposent notablement à l'introduction de la canule dans le septum d'un site implantable.

En conservant la forme biseautée pour réaliser le dispositif 1 de l'invention, l'homme du métier a donc dû vaincre des préjugés notables fondés sur le fait que la forme biseautée qui équipe habituellement les canules rigides est d'une utilisation à hauts risques de piqûres accidentelles.

De manière remarquable, afin de réduire le risque de piqûres accidentelles, l'extrémité distale biseautée 2D de la canule 2 est localement façonnée de manière à présenter une surface frontale 2G dont les génératrices d'enveloppe sont sensiblement parallèles à un plan sécant à l'axe longitudinal 2F de ladite canule 2.

Par l'expression "localement façonnée", on indique que l'enveloppe de la forme biseautée de l'extrémité distale de la canule n'est pas notablement affectée par le façonnage considéré.

De manière remarquable, en plus du fait qu'il est flexible conséquemment à la nature du matériau qui le constitue, le corps à paroi tubulaire 2A de la canule 2 comporte au moins un moyen M en vue de favoriser au moins sa flexion lorsqu'une action A sensiblement axiale est appliquée à son extrémité distale 2D en l'absence d'un élément rigidificateur placé dans son canal 2B.

Par cela, le risque de piqûres au moyen de la canule du dispositif de l'invention est encore réduit.

Dans une forme préférée de réalisation, le moyen M en vue de favoriser au moins la flexion de la canule 2 consiste en une précourbure de la canule 2.

Dans une autre forme de réalisation, le moyen M en vue de favoriser au moins la flexion de la canule 2 consiste en au moins un gaufrage localisé de la paroi de la canule 2.

Le corps rigide 5 destiné à être introduit dans le canal axial 2B de la canule 2 pour la rigidifier en vue de son introduction dans un milieu de réception 3A, 3B présente deux extrémités 5C, 5D dont :
- l'une 5C est dite proximale parce qu'elle est destinée à être placée au niveau de l'extrémité proximale 2C de la canule 2 et
- l'autre 5D est dite distale parce que, quant à elle, elle est destinée à être placée au niveau de l'extrémité distale 2D de la canule 2.

L'extrémité distale biseauté 5D du corps rigide 5 présente donc une face terminale 5E constituée dans un plan sensiblement sécant à un axe longitudinal 5F au dit corps rigide 5.

De manière remarquable :
- l'extrémité distale 5D du corps rigide 5 est biseautée sensiblement à la manière de l'extrémité distale 2D de la canule, c'est à dire de manière telle que les faces terminales 2E, 5E sont inclinées sensiblement d'une même valeur sur l'axe longitudinal 2F, 5F de l'élément 2, 5 qui la porte, et
- le dispositif comprend un moyen 6 de maintien de la canule 2 et du corps rigide 5 dans une position relative telle que les extrémités distales de la canule 2 et du corps rigide 5 sont séparés d'une distance D1 déterminée.

Le respect de ces particularités techniques permet de faciliter notablement l'introduction du dispositif dans le septum d'un site d'injection.

De manière encore remarquable, lorsque le milieu 3A, 3B, consiste en un site implanté 3B comprenant un septum 3C qui ferme une chambre d'injection 3D par une face 3E s'étendant à une distance déterminée D2 d'une face 3F opposée de la chambre d'injection 3D, la distance D1 qui extrémités distales de la canule 2 et du corps rigide 5 est inférieure à la dite distance D2 qui sépare les faces 3E, 3F en vis à vis du septum 3C et de la chambre d'injection 3D.

Par cela on est assuré que l'extrémité distale de la canule débouche dans la cavité interne du site 3B.

De manière encore remarquable, le dispositif comprend un mandrin 7 obturateur qui, réalisé en matériau flexible, est de diamètre sensiblement ajusté à celui du canal 2B de la canule 2 et, d'autre part, de longueur au moins égale à une fraction de la longueur de ladite canule 2.

Dans une forme de réalisation, le mandrin 7 en matériau flexible a un diamètre sensiblement inférieur à celui du canal 2B de la canule 2.

Selon une forme de réalisation, le mandrin 7 en matériau flexible a une longueur sensiblement égale à celle de la canule 2.

Le mandrin 7 comprend une extrémité proximale 7C équipée, d'une part, d'une butée 7E d'appui axial sur la face frontale de l'extrémité proximale 2C de la canule 2 et, d'autre part, d'une surface 7F de préhension, par exemple digitale, en vue de sa manipulation, c'est à dire de son introduction dans la canule 2 ou de son extraction de ladite canule 2.

Une fois engagé dans la canule 2, le mandrin 7 agit à la manière d'un obturateur du canal axial 2B.

Le mandrin flexible 7 permet donc d'obturer la canule sans affecter notablement la flexibilité de cette canule 2.

Une canule 2 équipé du mandrin flexible 7 peut donc être courbée pour être localement appliquée contre la peau d'un patient dans laquelle elle a été introduite.

L'application contre la peau du patient, de la partie recourbée de la canule, s'effectue, par exemple, au moyen d'un adhésif cutané A.

En se reportant au dessin, on voit une telle canule 2 qui, équipé d'un mandrin flexible 7, est engagée dans un site 3B implantée sous la peau 3A d'un patient (non représenté).

Ces particularités techniques permettent d'atténuer la gène imposée au patient, lorsque la canule 2 n'est temporairement pas utilisée pour la perfusion.

Le dispositif de l'invention peut donc être utilisé pour des perfusion séquentielles.

Une fois obturée avec le mandrin flexible 7, la canule 2 peut être recourbée et appliquée contre la peau du patient, ce qui permet de la dissimuler et d'obvier au risque d'accrochage.

Le mandrin flexible 7 permet d'éviter que la canule 2 soit obstruée par du sang coagulé ou du liquide de perfusion desséché.

De manière remarquable, la canule flexible 2 est pourvue d'au moins un moyen 8 de préhension digitale par pincement.

Dans une forme remarquable de réalisation, le moyen 8 de préhension digitale consiste en des ailettes 8A qui, réalisées en matériau flexible, sont au moins indirectement liées à l'extrémité proximale 2C de la canule 2.

Dans une forme préférée de réalisation, les ailettes 8A sont au moins indirectement articulées sur la canule 2 chacune autour d'un axe 8B approximativement parallèle à l'axe 2F du canal axial 2B de la canule 2.

Chacune des ailettes 8A présente deux faces opposées 8C et au moins l'une de ces faces est équipée d'un moyen 8D renforçant son adhérence contre la peau, tel un adhésif cutané.

## Revendications

1. Dispositif (1) à canule hypodermique (2), c'est à dire, un dispositif pour l'injection d'un liquide dans un site (3B), comprenant un septum (3C) qui ferme une chambre d'injection (3D), implanté dans un tissu vivant (3A), voire pour le prélèvement d'un liquide dans un tel site (3B),
la canule (2) consistant en un corps à paroi tubulaire (2A) qui, comprend notamment un canal axial (2B) et deux extrémités opposées (2C, 2D) dont l'une (2C), dite proximale, est destinée à être raccordée à un instrument (4) et, l'autre (2D) dite, distale, destinée à être engagée dans le site (3B),
le corps (2) tubulaire de la canule (2) étant réalisé dans un matériau choisi de manière que la canule (2) ait une flexibilité telle que son introduction précise dans un site (3B) ne puisse être obtenue qu'après rigidification au moyen d'un corps rigide (5) plein, tel une aiguille, introduit dans son canal axial (2B),
ce dispositif étant **CARACTERISE** en ce que :
- l'extrémité distale (2D) de la canule (2) est biseautée et localement façonnée de manière à présenter une surface frontale (2G) dont les génératrices d'enveloppe sont sensiblement parallèles à un plan sécant à l'axe longitudinal (2G) de ladite canule (2), et
- en plus du fait qu'il est flexible conséquemment à la nature du matériau qui le constitue, le corps à paroi tubulaire (2A) de la canule (2) comporte au moins un moyen (M) en vue de favoriser au moins sa flexion lorsqu'une action (A) sensiblement axiale est appliquée à son extrémité distale (2D) en l'absence d'un élément rigidificateur placé dans son canal (2B).

2. Dispositif selon la revendication 1 **caractérisé** en ce que le moyen (M) en vue de favoriser au moins la flexion de la canule (2) consiste en une précourbure de la canule (2).

3. Dispositif selon la revendication 2 **caractérisé** en ce que le moyen (M) en vue de favoriser au moins la flexion de la canule (2) consiste en au moins un gaufrage localisé de la paroi de la canule (2).

4. Dispositif selon l'une quelconque de revendications 1 à 3 et de type mettant en oeuvre un corps rigide (5) qui, destiné à être introduit dans le canal axial (2B) de la canule (2) pour la rigidifier en vue de son introduction dans un site (3B), présente deux extrémités (5C, 5D) dont :
- l'une (5C) est dite proximale, parce qu'elle est destinée à être placée au niveau de l'extrémité proximale (2C) de la canule (2) et
- l'autre (5D) est dite distale parce que, quant à elle est destinée à être placée au niveau de l'extrémité distale (2D) de la canule (2),
cette extrémité distale biseautée (5D) du corps rigide (5) présentant une face terminale (5E) constituée dans un plan sensiblement sécant à un axe longitudinal (5F) au dit corps rigide (5),
ce dispositif étant **caractérisé** en ce que :
- l'extrémité distale (5D) du corps rigide (5) est biseautée sensiblement à la manière de l'extrémité distale (2D) de la canule, c'est à dire de manière telle que les faces terminales (2E, 5E) sont inclinées sensiblement d'une même valeur sur l'axe longitudinal (2F, 5F) de l'élément (2, 5) qui la porte, et
- le dispositif comprend un moyen (6) de maintien de la canule (2) et du corps rigide (5) dans une position relative telle que les extrémités distales de la canule (2) et du corps rigide (5) sont séparés d'une distance (D1) déterminée.

5. Dispositif selon la revendication 4 **caractérisé** en ce que, lorsque le site (3B) consiste en un site implanté (3B) comprenant un septum (3C) qui ferme une chambre d'injection (3D) par une face (3E) s'étendant à une distance déterminée (D2) d'une face (3F) opposée de la chambre d'injection (3D), la distance (D1) qui sépare les extrémités distales de la canule (2) et du corps rigide (5) est inférieure à la dite distance (D2) qui sépare les faces (3E, 3F) en vis à vis du septum (3C) et de la chambre d'injection (3D).

6. Dispositif selon l'une quelconque des revendications 1 à 5 **caractérisé** en ce qu'il comprend un mandrin (7) obturateur qui, réalisé en matériau flexible, est de diamètre sensiblement ajusté à celui du canal (2B) de la canule (2) et, d'autre part, de longueur au moins égale à une fraction de la longueur de ladite canule (2).

7. Dispositif selon la revendication 6 **caractérisé** en ce que le mandrin (7) en matériau flexible a un diamètre sensiblement inférieur à celui du canal (2B) de la canule (2) et une longueur sensiblement égale à celle de la canule (2).

8. Dispositif selon l'une quelconque des revendications 1 à 7 et dont la canule flexible (2) est pourvue d'au moins un moyen (8) de préhension digitale par pincement, consistant en des ailettes (8A) qui, réalisées en matériau flexible, sont au moins indirectement liées à l'extrémité proximale (2C) de la canule (2) en étant articulées sur ladite canule (2) chacune autour d'un axe (8B) approximativement parallèle à l'axe (2F) du canal axial (2B) de la canule (2) **caractérisé** en ce que chacune des ailettes (8A) présente deux faces opposées (8C) et au moins l'une de ces faces est équipée d'un moyen (8D) renforçant son adhérence contre la peau.

## Claims

1. A hypodermic cannula (2) device (1), namely a device for injecting a liquid into a site (3B), comprising a septum (3C) closing an injection chamber (3D) and implanted into living tissue (3A), and also for taking a sample of a liquid from said site (3B),
the cannula (2) being formed by a body having a tubular wall (2A) and chiefly comprising an axial channel (2B) and two opposing ends (2C, 2D), the one, proximal end (2C) of which is intended for connection to an instrument (4), and the other, distal end (2D) of which is intended for insertion into the site (3B),
the tubular body (2A) of the cannula (2) being made of a material giving the cannula (2) flexibility such that it can only be precisely inserted into a site (3B) after rigidification by means of a solid rigid body (5), such as a needle, introduced into its axial channel (2B),
this device being characterised in that:
- the distal end (2D) of the cannula (2) is chamfered and locally shaped so as to have a front surface (2G) of which the exterior generatrices are substantially parallel to a plane intersecting the longitudinal axis (2F) of the said cannula (2), and
- in addition to the fact that it is flexible owing to the nature of the material of which it is made, the tubular body (2A) of the cannula (2) comprises at least one means (M) for at least assisting flexion of the cannula (2) when a substantially axial force (A) is applied to its distal end (2D) in the absence of a rigidifying member introduced into its channel (2B).

2. A device according to claim 1, characterised in that the means (M) for at least assisting flexion of the cannula (2) comprises pre-bending of the cannula (2).

3. A device according to claim 2, characterised in that the means (M) for at least assisting flexion of the cannula (2) comprises at least one localised embossment in the wall of the cannula (2).

4. A device according to any one of claims 1 to 3 and of the type utilising a rigid body (5) intended for introduction into the axial channel (2B) of the cannula (2) in order to rigidify the latter for its insertion into a site (3B) and having two ends (5C, 5D), of which:
- one (5C) is referred to as proximal because it is intended to be arranged at the level of the proximal end (2C) of the cannula (2), and
- the other (5D) is referred to as distal because it is intended to be arranged at the level of the distal end (2D) of the cannula (2),
this chamfered distal end (5D) of the rigid body (5) having an end surface (5E) formed in a plane substantially intersecting a longitudinal axis (5F) of the said rigid body (5),
this device being characterised in that:
- the distal end (5D) of the rigid body (5) is chamfered substantially in the manner of the distal end (2D) of the cannula, namely in a manner such that the end surfaces (2E, 5E) are inclined by substantially the same amount relative to the longitudinal axis (2F, 5F) of the member (2, 5), and
- the device comprises a means (6) for holding the cannula (2) and the rigid body (5) in a relative position such that the distal ends of the cannula (2) and the rigid body (5) are spaced apart by a predetermined distance (D1).

5. A device according to claim 4, characterised in that, when the site (3B) is an implanted site (3B) comprising a septum (3C) closing an injection chamber (3D) by means of a surface (3E) extending at a predetermined distance (D2) from an opposing surface (3F) of the injection chamber (3D), the distance (D1) separating the distal ends of the cannula (2) and the rigid body (5) is less than the distance (D2) separating the opposing surfaces (3E, 3F) of the septum (3C) and the injection chamber (3D).

6. A device according to any one of claims 1 to 5, characterised in that it comprises a sealing plug (7) made of flexible material and having a diameter substantially adapted to that of the channel (2B) of the cannula (2) and having a length at least equal to a portion of the length of the said cannula (2).

7. A device according to claim 6, characterised in that the plug (7) of flexible material has a diameter substantially smaller than that of the channel (2B) of the cannula (2) and a length substantially equal to that of the cannula (2).

8. A device according to any one of claims 1 to 7 and of which the flexible cannula (2) is provided with at least one means (8) enabling the cannula (2) to be gripped by the fingers, comprising wings (8A) made of a flexible material and at least indirectly connected to the proximal end (2C) of the cannula (2), each being articulated on the said cannula (2) about an axis (8B) approximately parallel to the axis (2F) of the axial channel (2B) of the cannula (2), characterised in that each of the wings (8A) has two opposing surfaces (8C) and at least one of these surfaces is provided with a means (8D) increasing its adhesion to the skin.

## Patentansprüche

1. Vorrichtung (1) mit Kanüle (2) zur subkutanen Injektion, d.h. einer Vorrichtung zum Einspritzen einer Flüssigkeit in eine Injektionsstelle (3B), welche eine Trennwand (3C) aufweist, die eine Injektionskammer (3D) verschließt, die in Lebendes Gewebe (3A) implantiert ist, und zwar zur Entnahme einer Flüssigkeit in einer solchen Injektionsstelle (3B),
wobei die Kanüle (2) aus einem Korpus mit rohrförmiger Wandung (2A) besteht, welche vornehmlich einen axial verlaufenden Kanal (2B) und zwei sich gegenüberliegende Enden (2C, 2D) aufweist, von denen das eine (2C), das sogenannte proximale Ende, zum Anschluß an ein Instrument (4) und das andere (2D), das sogenannte distale Ende, zum Eingriff mit der Injektionsstelle (3B) bestimmt ist,
wobei der rohrförmige Korpus (2) der Kanüle aus einem Werkstoff gefertigt ist, der so gewählt ist, daß die Kanüle (2) eine solche Biegsamkeit besitzt, daß ihre exakte Einführung in eine Injektionsstelle (3B) erst nach Versteifung mit Hilfe eines massiven starren Körper (5) wie beispielsweise einer Nadel erfolgen kann, die in ihren axialen Kanal (2B) eingeführt ist,
dadurch **GEKENNZEICHNET**, daß:
- die Kanüle (2) abgeschrägt und örtlich begrenzt so geformt ist, daß sie eine Vorderfläche (2G) aufweist, deren Mantellinien im wesentlichen parallel zu einer Ebene verlaufen, die sich mit der Längsachse (2G) der Kanüle (2) schneidet, und
- der Korpus der rohrförmigen Wandung (2A) der Kanüle (2) neben seiner biegsamen Ausbildung infolge der Art des Werkstoffs, aus dem er besteht, mindestens eine Einrichtung (M) aufweist, welche zumindest dessen Biegung begünstigt, wenn auf das distale Ende (2D) der Kanüle eine im wesentlichen in axialer Richtung wirksame Kraft (A) aufgebracht wird und dabei ein Versteifungselement fehlt, das sonst in dem Kanal (2B) der Kanüle angeordnet ist.

2. Vorrichtung nach Anspruch 1, dadurch **gekennzeichnet**, daß die Einrichtung (M) zum Begünstigen mindestens der Biegung der Kanüle (2) aus einer Vorkrümmung der Kanüle (2) besteht.

3. Vorrichtung nach Anspruch 2, dadurch **gekennzeichnet**, daß die Einrichtung (M) zum Begünstigen mindestens der Biegung der Kanüle (2) aus mindestens einer örtlich begrenzten Prägung der Wandung der Kanüle (2) besteht.

4. Vorrichtung nach einem der Ansprüche 1 bis 3 von der Art, bei welcher ein starrer Korpus (5) zum Einsatz kommt, welcher zur Einführung in den axial verlaufenden Kanal (2B) der Kanüle (2) zu deren Aussteifung zum Einführen derselben in eine Injektionsstelle (3B) bestimmt ist und zwei Enden (5C, 5D) aufweist. wovon:
- das eine (5C) das sogenannte proximale Ende ist, da es zur Anordnung in Höhe des Promillen Endes (2C) der Kanüle (2) bestimmt ist, und
das andere (5D) das sogenannte distale Ende ist, da es seinerseits zur Anordnung in Höhe des distalen Endes (2D) der Kanüle (2) bestimmt ist,
dadurch **gekennzeichnet**, daß:
- das distale Ende (5D) des starren Korpus (5) im wesentlichen in gleicher Weise wie das distale Ende (2D) der Kanüle und somit in der Weise abgeschrägt ist, daß die Endflächen (2E, 5E) im wesentlichen um den gleichen Betrag gegenüber der Längsachse (2F, 5F) des Elements (2, 5) geneigt sind, welches diese trägt, und
- die Vorrichtung eine Einrichtung (6) zum Halten der Kanüle (2) und des starren Korpus (5) in einer solchen relativen Position aufweist, daß die distalen Enden der Kanüle (2) und des starren Korpus (5) um einen vorgegebenen Abstand (D1) voneinander getrennt sind.

5. Vorrichtung nach Anspruch 4, dadurch **gekennzeichnet**, daß dann, wenn die Injektionsstelle (3B) aus einer implantierten Injektionsstelle (3B) besteht, welche eine Trennwand (3C) zum Verschließen einer Injektionskammer (3D) über eine Fläche (3E) aufweist, die sich in einem vorgegebenen Abstand (D2) von einer der Injektionskammer (3D) gegenüber liegenden Fläche (3F) aus erstreckt, der Abstand (D1), der die distalen Enden der Kanüle (2) und des starren Korpus (5) trennt, kleiner ist als der Abstand (D2), welcher die Flächen (3E, 3F) gegenüber der Trennwand (3C) und der Injektionskammer (3D) trennt.

6. Vorrichtung nach einem der Ansprüche 1 bis 5, dadurch **gekennzeichnet**, daß sie einen Verschlußdorn (7) aufweist, der aus einem biegsamen Werkstoff hergestellt ist und einen Durchmesser aufweist, der im wesentlichen auf den Durchmesser des Kanals (2B) der Kanüle (2) eingestellt ist, und zum anderen eine Länge besitzt, die mindestens gleich einem Bruchteil der Länge der Kanüle (2) ist.

7. Vorrichtung nach Anspruch 6, dadurch **gekennzeichnet**, daß der Dorn (7) aus biegsamem Werkstoff einen etwas kleineren Durchmesser als der Kanal (2B) der Kanüle (2) und eine Länge besitzt, die im wesentlichen gleich der Länge der Kanüle (2) ist.

8. Vorrichtung nach einem der Ansprüche 1 bis 7, bei welcher die biegsame Kanüle (2) mit mindestens einer Einrichtung (8) zum Erfassen durch Festklemmen mit den Fingern versehen ist, die aus Flügeln (8A) besteht, die aus biegsamem Werkstoff hergestellt sind und zumindest indirekt mit dem proximalen Ende (2C) der Kanüle (2) verbunden und dabei auf der Kanüle (2) jeweils um eine Achse (8B) angelenkt sind, welche parallel zur Achse (2F) des axialen Kanals (2B) der Kanüle (2) verläuft, dadurch **gekennzeichnet**, daß jeder der Flügel (8A) zwei gegenüberliegende Flächen (BC) aufweist und mindestens eine dieser Flächen mit einer. Einrichtung (8D) versehen ist, welche ihre Anlage gegen die Haut verstärkt.
